# EUROPEAN PATENT APPLICATION

(11) **EP 0 969 016 A2**
(43) Date of publication of application: **05.01.2000**
(21) Application number: 99110184.1
(22) Date of filing: 14.04.1993
(51) Int. Cl.: C07K 14/605, A61K 38/26

(54) **Glucagon-like peptide and insulinotropin derivates**

(30) Priority: 15.06.1992 US 899073
(62) Divisional of application: 93909505.5
(71) Applicant: SCIOS INC., Mountain View, CA 94043 (US)
(72) Inventor: Andrews, Glenn C., Waterford, CT 06385 (US); Daumy, Gaston O., Gales Ferry, CT 06335 (US); Francoeur, Michael L., Hillsborough, CA 94010 (US); Larson, Eric R., Mystic, CT 06355 (US)
(74) Representative: White, Martin Paul

(57) **Abstract**

This invention relates to derivatives of glucagon-like peptide 1 (GLP-1), truncated GLP-1, insulinotropin and truncated insulinotropin which have a pl of about 4.0 or less or a pl of about 7.0 or greater. The derivatives of GLP-1, truncated GLP-1, insulinotropin and truncated insulinotropin within the scope of this invention are particularly suited for delivery to a mammal by iontophoresis. This invention also relates to methods for enhancing insulin action in a mammal with said derivatives and to pharmaceutical compositions comprising said derivatives. Further still, this invention relates to new uses of certain known derivatives of insulinotropin and truncated insulinotropin to enhance insulin action in a mammal by iontophoretic administration of such derivatives.

## Description

### Field of the invention

This invention relates to derivatives of glucagon-like peptide 1(GLP-1),truncated GLP-1, insulinotropin and truncated insulinotropin. More specifically, this invention relates to derivatives of GLP-1, truncated GLP-1, insulinotropin and truncated insulinotropin, and tile pharmaceutically acceptable salts thereof, which have a pl of about 4.0 or less or a pl of about 7.0 or greater. The derivatives of GLP-1,truncated GLP-1, insulinotropin and truncated insulinotropin within the scope of this invention are particularly suited for delivery to a mammal by iontophoresis. The derivatives of this invention have insulinotropic activity and are useful for enhancing insulin action in a mammal. The methods of treatment of this invention comprise administering to a mammal an effective amount of a derivative of GLP-1, truncated GLP-1, insulinotropin or truncated insulinotropin. Further, this invention relates to pharmaceutical compositions comprising said derivatives of GLP-1, truncated GLP-1, insulinotropin and truncated insulinotropin. Further still, this invention relates to new uses of certain known derivatives of insulinotropin and truncated insulinotropin to enhance insulin action in a mammal by iontophoretic administration of such derivatives.

### Background Art

The amino add sequence of GLP-1 is known as
His-Asp-Glu-Phe-Glu-Arg-His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 1).

GLP-1 is disclosed by Lopez, L. C., et al., P.N.A.S.. USA 80:5485-5489 (1983); Bell, G. I., et al, Nature 302:716-718 (1983); Heinrick, G., et al, Endocrinol. 115:2176-2181 (1984) and Ghiglione, M., et al., Diabetologia 27:599-600 (1984).

GLP-1 is known to be naturally processed through conversion to a 31-amino acid peptide having amino acids 7-37 of GLP-1 (7-37). This processing reportedly occurs in the pancreas and intestine. The 7-37 peptide, herein referred to alternatively as insulinotropin, is a hormone that has insulinotropic activity.

Insulinotropin has the following amino acid sequence:
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 2).

Insulinotropin, certain derivatives thereof and the use thereof to treat Diabetes mellitus in a mammal are disclosed in PCT/US87/01005 (WO87/06941), published November 19, 1987. The teachings thereof are incorporated herein by reference. Derivatives of insulinotropin disclosed in PCT/US87/01005 include polypeptides which contain or lack one or more amino acids that may not be present in the naturally-occurring sequence. Further derivatives of insulinotropin disclosed in PCT/US87/01005 include certain C-terminal salts, esters and amides where the salts and esters are defined as OM where M is a pharmaceutically acceptable cation or a lower (C₁-C₆)branched or unbranched alkyl group and the amides are defined as -NR²R³ where R² and R³ are the same or different and are selected from the group consisting of hydrogen and a lower (C₁-C₆)branched or unbranched alkyl group.

Certain other polypeptides, herein alternatively referred to as truncated insulinotropin, having insulinotropic activity and the derivatives thereof are disclosed in PCT/US89/01121 (WO 90/11296). Those polypeptides, referred to therein as GLP-1 (7-36), GLP-1 (7-35) and GLP-1 (7-34) have the following amino acid sequences. respectively.
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg (SEQUENCE ID NO: 3);
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly(SEQUENCE ID NO: 4); and
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys (SEQUENCE ID NO: 5).

Derivatives of the polypeptides disclosed in PCT/US89/01121 include polypeptides having inconsequential amino acid substitutions, or additional amino acids to enhance coupling to carrier protein or to enhance the insulinotropic effect thereof. Further derivatives of insulinotropin disclosed In PCT/US89/01121 include certain C-terminal salts, esters and amides where the salts and esters are defined as OM where M is a pharmaceutically acceptable cation or a lower branched or unbranched alkyl group and the amides are defined as -NR²R³ where R² and R³ are the same or different and are selected from the group consisting of hydrogen and a lower branched or unbranched alkyl group.

Delivery of therapeutically effective polypeptides to a mammal present certain problems well known to those skilled in the art. Oral administration of a polypeptide, without some form of delivery device, will generally be unsuccessful due to the low inherent permeability of the intestine and other processes such as chemical degradation in the stomach and intestine. Transdermal administration of polypeptides affords the possibility of providing therapeutically effective polypeptides to a mammal without subjecting the polypeptides to degradation in the gut. Various approaches for transdermal administration of pharmaceutically active compounds are known to those skilled in the art. One such approach for transdermal administration Is a method known to those skilled in the art as iontophoresis.

Iontophoresis involves the application of a potential electrical gradient across the skin in conjunction with the surface co-application of therapeutic agents. To accomplish this, two electrodes are required in addition to a drug reservoir and a power source. Various type of iontophoretic devices are described by Tyle, P., Pharmaceutical Research 3:318-326 (1986). An example of an electrode for use in iontophoresis is disclosed in U.S. 4,950,229, the teachings of which are incorporated herein by reference. The result of iontophoresis is transport of therapeutic agents across the skin to either local or systemic sites. Further, iontophoresis is known to comprise the application of different voltage patterns including such methods as electroporation or pulsed current techniques.

A low level of electrical current has been used to transdermally administer leuprolide, a synthetic 9 amino add leutinizing hormone releasing hormone analog. Meyer, B. R., et al., Clin. Pharmacol. Ther. 44:607-612 (1988). A study on the iontophoretic delivery of insulin to rats has been reported. Siddiqui, O., et al. J. Pharmaceutical Sciences 76:341-345 (1987). Studies on transdermal iontophoresis of gonadotropin releasing hormone and thyrotropin releasing hormone have been reported. Miller, L. L, et al., J. Pharmaceutical Sciences 79:490-493 (1990) and Burnette, R. R., et al., J. Pharmaceutical Sciences 75:738-743 (1986). Ethanol, has been disclosed as enhancing iontophoretic transdermal delivery of leuprolide and CCK-8 (cholecystokinin-8) analog. Srinivasan, V. et al., J. Pharmaceutical Sciences 79:588-591 (1990).

### Disclosure of the Invention

This invention relates to polypeptide derivatives of glucagon-like peptide 1 (GLP-1) and truncated GLP-1 comprising the primary structure
H₂N-W-COOH
wherein W is an amino acid sequence selected from the group consisting of
His-Asp-Glu-Phe-Glu-Arg-His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 1) and
His-Asp-Glu-Phe-Glu-Arg-His-Ala-Glu-Gly-Thr-Phe-Thr-Ser-Asp-Val-Ser-Ser-Tyr-Lau-Glu-Gly-Gln-Ala-Ala-Lys-Glu-Phe-Ile-Ala-Trp-Leu-Val-Lys-Gly-Arg (SEQUENCE ID NO: 6);
and the pharmaceutically-acceptable salts thereof wherein the derivative has a pl of about 4.0 or less, or a pl of about 7.0 or greater and when processed in a mammal results in a polypeptide derivative having insulinotropic activity.

This invention further relates to polypeptide derivatives of GLP-1 and truncated GLP-1 described above comprising the primary structure
H₂N-W-(X)ₘ-(Y)ₙ-Z
and the pharmaceutically-acceptable salts thereof wherein W is as defined above; m is zero or one; n is zero or one; X is a basic or neutral L-amino add residue; Y is a basic or neutral L-amino acid residue; and Z is CO₂R¹ or CONR²R³ wherein R¹ is H or (C₁-C₆) straight or branched-chain alkyl when m is one, n is zero and X is a basic L-amino acid residue, or m is zero, n is one and Y is a basic L-amino acid residue, or m and n are both one and one or both of X and Y are a basic L-amino acid residue; R¹ is (C₁-C₆)straight or branched-chain alkyl when m and n are both zero, or m is one, n is zero and X is a neutral L-amino acid residue, or m is zero, n is one and Y is a neutral L-amino acid residue, or m and n are both one and both X and Y are a neutral L-amino acid residue; and R² and R³ are each, independently, H or (C₁-C₆)straight or branched-chain alkyl.

Further still, this invention relates to derivatives of polypeptides comprising the primary structure
H₂N-R-COOH
wherein R is an amino acid sequence selected from the group consisting of
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 2),
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg (SEQUENCE ID NO: 3),
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly (SEQUENCE ID NO: 4)and
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys (SEQUENCE ID NO: 5);
and the pharmaceutically-acceptable salts thereof wherein the derivative has a pl of about 4.0 or less, or a pl of about 7.0 or greater and having insulinotropic activity, provided that said derivative is not a C-terminal (C₁-C₆)straight or branched-chain alkyl ester and provided further that said derivative is not a C-terminal carboxamide of the formula CONR²R³ wherein R² and R³ are each, independently, H or (C₁-C₆)straight or branched-chain alkyl.

This invention also relates to derivatives of the polypeptides described immediately above comprising the primary structure
H₂N-R-X-(Y)ₙ-Z
and the pharmaceutically-acceptable salts thereof wherein R is as described above; n is zero or one; X is a basic or neutral L-amino acid residue; Y Is a basic or neutral L-amino acid residue; and Z is CO₂R¹ or CONR²R³ wherein R¹ is H or (C₁-C₆) straight or branched-chain alkyl when n is zero and X is a basic L-amino acid residue, or n is one and one or both of X and Y are a basic L-amino acid residue; R¹ is (C₁-C₆)straight or branched-chain alkyl when n is zero and X is a neutral L-amino acid residue, or n is one and both X and Y are a neutral L-amino acid residue; and R² and R³ are each, independently, H or (C₁-C₆)straight or branched-chain alkyl.

Preferred derivatives of this invention are those having a pl of about 8.5 or greater. Still other preferred derivatives of such polypeptides are those wherein R is
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 2). Even more preferred are such derivatives wherein R is
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 2):
n is zero and X is Arg or n is one and X and Y are each Arg. Still more preferred derivatives are such preferred derivatives wherein Z is CO₂R¹ and R¹ is H or Z is CONR²R³ and R² and R³ are each H.

This invention further relates to methods of enhancing insulin action in a mammal which methods comprise administering to said mammal an effective amount of a derivative according to this invention. A preferred method of enhancing insulin action according to this invention comprises treatment of Type II diabetes. A preferred method of administration of such derivatives is iontophoretic transdermal administration.

Still further yet, this invention relates to a method of enhancing insulin action in a mammal which comprises iontophoretically administering to said mammal an effective amount of a derivative of a polypeptide comprising the primary structure
H₂N-R-(X)ₘ-(Y)ₙ-Z
wherein R is an amino acid sequence selected from the group consisting of
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 2),
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg (SEQUENCE ID NO: 3),
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly (SEQUENCE ID NO: 4) and
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys (SEQUENCE ID NO: 5);
and the pharmaceutically-acceptable salts thereof wherein m is zero or one; n is zero or one; X is a basic or neutral L-amino acid residue; Y is a basic or neutral L-amino acid residue; and Z is CO₂R¹ or CONR²R³ wherein R¹ is H or (C₁-C₆) straight or branched-chain alkyl when m is one, n is zero and X is a basic L-amino acid residue, or m is zero, n is one and Y is a basic L-amino add residue, or m and n are both one and one or both of X and Y are a basic L-amino add residue; R¹ is (C₁-C₆)straight or branched-chain alkyl when m and n are both zero, or m is one, n is zero and X is a neutral L-amino acid residue, or m is zero, n is one and Y is a neutral L-amino acid residue or m and n are both one and both X and Y are a neutral L-amino acid residue; and R² and R³ are each, independently, H or (C₁-C₆)straight or branched-chain alkyl.

The derivatives of this invention can also be used in connection with other therapies such as other anti-diabetic agents (e.g., sulfonylureas).

A preferred method of iontophoretically administering a derivative of a polypeptide as described immediately above comprises iontophoretically administering said derivative wherein R is
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 2):
m is zero and n is zero,
with a still more preferred method comprising said preferred method wherein Z is CONR²R³ and R² and R³ are each hydrogen.

This invention further relates to pharmaceutical compositions comprising derivatives of polypeptides according to this invention. Such pharmaceutical compositions are useful in enhancing insulin action in a mammal. Thus, the pharmaceutical compositions of this invention are particularly suited for treatment of certain diabetic conditions such as Type II diabetes.

### Detailed Description

The term 'derivative', as used throughout this Specification and the appendant claims, includes, but is not limited to, polypeptides comprising the primary structure shown, wherein one or more L-amino acids are included at the C-terminus thereof; wherein the C-terminal carboxyl group forms an ester with a (C₁-C₆)straight or branched-chain alkyl group; wherein the C-terminal carboxyl group forms a carboxamide or substituted carboxamide; wherein the acidic amino add residues (Asp and/or Glu) form an ester or carboxamide; and combinations thereof.

The term "basic L-amino acid residue", as used throughout the Specification and the appendant claims, includes, but is not limited to, the common amino acids Lys, Arg and His.

The term "neutral L-amino acid residue", as used throughout this Specification and the appendant claims, includes, but is not limited to, Ala, Val, Leu, Ile, Pro, Met, Phe,Trp, Gly, Ser,Thr, Cys,Tyr, Asn and Glu. While Gly is not technically, an L-amino acid residue due to the presence of only hydrogen at the α-carbon in addition to the carboxyl and amino groups, it is referred to herein as an L-amino acid for the sake of simplicity.

The foregoing classification of L-amino acid residues as basic or neutral is based upon the net charge of the corresponding amino acid at pH 7.0.

As used throughout this Specification and the appendant claims, the "term pl" refers to the theoretical pl which is calculated using commercial software known as PCGENE (IntelliGenetics, Inc., 700 East El Camino Real, Mountain View, CA 94040).

Included within the scope of this invention are polypeptides having homology to the polypeptides described above, which homology is sufficient to impart insulinotropic activity to such polypeptides. Also included within the scope of this invention are variants of the polypeptides described above, which variants comprise inconsequential amino acid substitutions and have insulinotropic activity.

The phrase "enhancing insulin action", as used throughout this Specification and the appendant claims, includes, but is not limited to, one or more of increasing insulin synthesis, increasing insulin secretion, increasing glucose uptake by muscle and fat and decreasing glucose production by the liver.

The polypeptides of this invention are prepared by various methods well known to those skilled in the art. For example, the polypeptides can be synthesized using automated peptide synthesizers such as an Applied Biosystems (ABI)430A solid phase peptide synthesizer. Alternatively, the polypeptides wherein Z is CO₂H of this invention can be prepared using recombinant DNA technology wherein a DNA sequence coding for the polypeptide is operably linked to an expression vector and used to transform an appropriate host cell. The transformed host cell is then cultured under conditions whereby the polypeptide will be expressed. The polypeptide is then recovered from the culture. Further still, a combination of synthesis and recombinant DNA techniques can be employed to produce the amide and ester derivatives of this invention and/or to produce fragments of the desired polypeptide which are then joined by methods well known to those skilled in the art.

Derivatives of the polypeptides according to this invention are prepared by methods well known to those skilled in the art. For example, C-terminal alkyl ester derivatives of the polypeptides of this invention are prepared by reacting the desired (C₁-C₆)alkanol with the desired polypeptide in the presence of a catalytic acid such as HCL. Appropriate reaction conditions for such alkyl ester formation include a reaction temperature of about 50°C and reaction times of about 1 hour to about 3 hours. Similarly, derivatives of the polypeptides of this invention comprising (C₁-C₆)alkyl esters of the Asp and/or Glu residues within the polypeptide can be so formed.

Preparation of carboxamide derivatives of the polypeptides of this invention are also prepared by solid phase peptide synthesis methods well known to those skilled in the art. For example, see, Solid Phase Peptide Synthesis, Stewart, J. M. et al., Pierce Chem. Co. Press, 1984. When derivatives of the polypeptides according to this invention having a pl of about 4.0 or less are desired, such derivatives can be prepared by various methods well known to those skilled in the art. For example, deamidation of a glutamine residue to produce a glutamic acid residue, alkylation or amidation of free amino groups at the N-terminus and/or at the epsilon amino groups of lysine residues, or a combination thereof will result in derivatives having lower pl values. To decrease the pl of a derivative of insulinotropin to less than about 3.89, modification of any two of the available amino groups, or modification of at least one amino group and deamidation of the glutamine are necessary. Deamidation of glutamine residues is readily accomplished by suspension of the desired polypeptide of this invention in water at a pH greater than 8 for a period of several hours.

For example, depending on the pH of the reaction, acetylation of insulinotropin under basic conditions can afford the derivative wherein both amidation of the N-terminal amino group and both of the epsilon amino groups has occurred. The result is a derivative with a theoretical pl of 3.61. Alternatively, N-terminal acetylation combined with deamidation of the single glutamine residue of insulinotropin to a glutamic acid residue yields a derivative with a theoretical pl of 4.11.

As an alternative method for reducing the pl, a cyclic anhydride can be reacted with a polypeptide to block a basic residue and introduce an acidic residue. By way of example and not of limitation, a solution of insulinotropin (SEQUENCE ID NO.: 2) in DMF in the presence of 8 equivalents of triethylamine and 8 equivalents of succinic anhydride affords the N-succinate derivative of insulinotropin at the N-terminus and the Lys₂₀ and Lys₂₈ residues thereof.

Alternatively, or in combination-with the above, derivatives of the polypeptides of this invention can be prepared by modifying the DNA coding sequence for such polypeptide so that a basic amino acid residue is replaced with an acidic or neutral amino acid residue, or a neutral amino acid residue is replaced with an acidic amino acid residue. Such changes in polypeptide primary sequence can also be accomplished by direct synthesis of the derivative. Such methods are well known to those skilled in the art. Of course, such derivatives, to be useful in the practice of this invention, must achieve an insulinotropic effect.

The insulinotropic activity of a polypeptide derivative according to this invention, wherein said polypeptide does not include amino acids 1-6 of GLP-1 or truncated GLP-1, is determined as follows.

Pancreatic islets are isolated from pancreatic tissue from normal rats by a modification of the method of Lacy, P. E., et al., Diabetes, 16:35-39(1967) in which the collagenase digest of pancreatic tissue is separated on a Ficoll gradient (27%, 23%, 20.5% and 11% in Hanks' balanced salt solution, pH 7.4). The islets are collected from the 20.5%/11% interface, washed and handpicked free of exocrine and other tissue under a stereomicroscope. The islets are incubated overnight in RPMI 1640 medium supplemented with 10% fetal bovine serum and containing 11 mM glucose at 37°C and 95% air/5% CO₂. The islets are then transferred to RPMI 1640 medium supplemented with 10% fetal bovine serum and containing 5.6 mM glucose. The islets are incubated for 60 minutes at 37°C, 95% air/5% CO₂. The polypeptide derivative to be studied is prepared at 1 nM and 10 nM concentrations in RPMI medium containing 10% fetal bovine serum and 16.7 mM glucose. About 8 to 10 isolated islets are then transferred by pipete to a total volume of 250 µl of the polypeptide derivative containing medium in 96 well microtiter dishes. The islets are incubated in the presence of the polypeptide derivative at 37°C, 95% air/5% CO₂ for 90 minutes. Then, aliquots of islet-free medium are collected and 100 µl thereof are assayed for the amount of insulin present by radioimmunoassay using an Equate Insulin RIA Kit (Binax, Inc., Portland, ME).

Pharmaceutical compositions comprising polypeptide derivatives according to this invention can be prepared according to methods well known to those skilled in the art. For example, the polypeptide derivatives can be combined with a pharmaceutically acceptable diluent or carrier. When the polypeptide derivatives of this invention are to be administered intravenously, intramuscularly or subcutaneously, appropriate sterile diluent is employed as is well known in the art. Such pharmaceutical compositions will comprise a sufficient amount of the polypeptide derivative so that an appropriate dosage, as hereinbelow described, can be administered over an appropriate period of time.

For iontophoretic delivery of a polypeptide derivative according to this invention, various compositions can be prepared. The polypeptide derivative can be included in a solution or as part of a gel or foam. It is preferable, however, that the polypeptide derivative in such composition have the same or approximately the same charge as the electrode in the drug reservoir of the iontophoretic device to be employed. The charge of the derivative can be controlled, for example, by the use of an appropriate buffer. When using a buffer, it is preferable to employ a buffer which has a charge opposite to that of the particular polypeptide derivative to be administered. Alternatively, the polypeptide derivative may act as its own "buffer" if the appropriate salt form thereof is employed. Variables in such compositions include the concentration of the polypeptide derivative, the buffer concentration when present, the ionic strength of the composition and the nonaqueous cosolvents. In general, to achieve the highest transport efficiency by iontophoresis with such compositions, it is preferable to minimize the concentration of all ionic species except the polypeptide derivative in such compositions. Adjustment of such concentrations are within the skill of those who practice in the art, enabled by the disclosure herein.

A variety of iontophoretic devices are known. Various electrode materials for use with such devices are known and available. Such electrodes include those made of platinum or silver-sliver chloride. The differences among electrodes are known to be associated with certain performance nuances. For example, use of platinum electrodes cause hydrolysis leading to the liberation of hydrogen ions and subsequent changes in pH. Changes in pH, in turn, can influence the ionization state of the polypeptide derivative and, thus, the resulting iontophoretic transport thereof. Silver-silver chloride electrodes, on the other hand, do not hydrolyze water when used with iontophoretic devices. Such silver-silver chloride electrodes, however, require the presence of chloride ions which may compete for current-induced transport across the skin. Choice of the appropriate electrode for use in iontophoretic administration of the polypeptide derivatives according to this invention is within the skill of those who practice in the art, enabled by the disclosure herein.

In addition, the methods for evaluating iontophoretic delivery using a porcine skin flap, as described by Riviere, J. E., et al., J. Toxicol.-Cut. & OcularToxicol. 8:493-504 (1989-1990) can be employed with the polypeptide derivatives and compositions of this invention.

Dosages effective in treatment of adult onset diabetes will range from about 1 pg/kg to 1000 µg/kg per day when a polypeptide derivative of this invention is administered intravenously, intramuscularly or subcutaneously. A preferred dosage range for intravenous infusion during and between meals is about 4 to 10 ng/kg/min or about 0.6 to 1.4 µg/day based on a 100 kg patient. It is to be appreciated, however, that dosages outside of that range are possible and are also within the scope of this invention. The appropriate dosage can and will be determined by the prescribing physician and will be a result of the severity of the condition being treated as well as the response achieved with the derivative being administered and the age, weight, sex and medical history of the patent. For iontophoretic administration of a polypeptide derivative according to this invention, a dosage range of about 500 to 1000 µg/per day. Here, too, dosages outside of that range are possible and are within the scope of this invention.

### EXAMPLE 1

### H₂N-R-X-(Y)ₙ-Z wherein n is zero; X is arg; Z is CONR²R³ where R² and R³ are H; and R is His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 2)

The title peptide was synthesized starting with a p-methylbenzhydrylamine (HCl salt) resin using an Applied Biosystems (ABI) 430A solid phase peptide synthesizer [mfr, address] using ABI Version 1.40 of the N-methylpyrrolidone/hydroxybenzotriazole t-BOC cycles. The end cycle was selected to remove the ultimate t-BOC protecting group at the completion of the synthesis. The following amino add side chain protection was used: Arg (Tos), Lys (Cl-Z), Trp (CHO), Glu (OCyHex), Tyr (Br-Z), Ser (Bzl), Thr (Bzl), Asp (OCyHex) and His (BOM). The synthesis cycles used were those provided by ABI with the following modifications: the delivery time of hydroxybenzotriazole to the measuring loop was increased from 6 seconds to 10 seconds to assure reproducible and proper delivery thereof: and the delivery time of hydroxybenzotriazole to the activator vessel was increased from 12 seconds to 18 seconds. In order to prevent clogs in the instrument caused by vapors generated by the acetic anhydride reagent bottle, the reaction vessel cycle was modified to pressurize the associated valve block alter each delivery of acetic anhydride. The ABOC11 activator cycle was used for the activation of Glu in place of the normally used ABOC12. After final removal of the N-terminal t-BOC group, a total of 2.65 g of peptide-resin was obtained. Then, the formyl protecting group was removed from the Trp residue by treatment of the peptide-resin for 1 hour by gently shaking in a solution containing 2 ml H₂O, 2 ml of 70% - ethanolamine in methanol and 16 ml dimethylformamide. Then, the resin was filtered, washed successively with dimethylformamide (3 x 10 ml), methanol (3 x 10 ml) and dichloromothane (3 x 10 ml). The washed resin was dried in vacuo to yield 2.48 g of resin.

To remove the peptide from the resin, 997 mg of the dried resin was treated with liquid hydrogen fluoride/10% m-cresol at 0°C for 1 hour. Then, the hydrogenfluoride was removed by evaporation and the peptide was taken up in trifluoroacetic acid. The peptide was then precipitated using ethyl ether to yield 403 mg of peptide as a white solid. Analytical HPLC data suggested incomplete removal of the Trp formyl protecting group in the peptide. To remove the remaining formyl protecting groups, 40 mg of the peptide was dissolved in a mixture of 3.6 ml H₂O and 0.4 ml of 70% ethanolamine in methanol. The resulting solution was allowed to sit for 30 minutes at room temperature. Then, 0.35 ml of trifluoroacetic acid was added and the precipitate was collected by centrifugation (14,000 rpm, 5 min.). The collected precipitate was dissolved in 4 ml 6M guanidine-HCl and chromatographed by preparative reverse phase HPLC on a 1 inch VYDAC C18 column using a gradient system of 100% → 40%A, 0% → 60%B over a period of 60 min, at a flow rate of 10 ml/min. (A is 0.1% trifluoroacetic acid/95% H₂O/5% CH₃CN and B is CH₃CN) to afford 10.5 mg of the title peptide.
FAB MS: 3511.4 Da (parent + H predicted: 3511 Da)

### EXAMPLE 2

### H₂N-R-X-(Y)ₙ-Z wherein n is zero; X is arg; Z is CO₂R¹ where R¹ is H; and R is His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 2)

Using the procedure described in Example 1, modified to synthesize the title peptide, a total of 2.0 g of peptide resin was prepared. Treatment of 900 mg of the peptide resin with hydrogen fluoride according to the procedure of Example 1 yielded 340 mg of peptide after precipitation of the trifluoroacetic acid solution from ethyl ether. Then, as in Example 1, 40 mg of the peptide was treated in aqueous ethanolamine, acidified, precipitated and chromatographed to afford 10 mg of the title peptide.
FAB MS: 3511.9 Da (parent + H predicted: 3512 Da)

### EXAMPLE 3

### H₂N-R-X-(Y)ₙ-Z wherein n is one; X is arg; Y is arg; Z is CONR²R³ where R² and R³ are H; and R is His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 2)

Using the procedure described in Example 1, modified to synthesize the title peptide; a total of 1.3 g of peptide resin was prepared. Treatment of 1.3 g of the peptide resin with hydrogen fluoride according to the procedure of Example 1 yielded 671 mg of the peptide after precipitation of the trifluoroacetic acid solution from ethyl ether. Then, as in Example 1, 7 mg of the peptide was treated in aqueous ethanolamine, acidified, precipitate and chromatographed to afford 4.2 mg of the title peptide.
FAB MS: 3667.8 Da (parent + H predicted: 3667.81 Da)

### EXAMPLE 4

### H₂N-R-X-(Y)ₙ-Z wherein n is one; X is arg; Y is erg; Z is CO₂R¹ where R¹ is H; and R is His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 2)

Using the procedure described in Example 1, modified to synthesize the title peptide; a total of 2.66 g of peptide resin was prepared. Treatment of 1.1 g of the peptide resin with hydrogen fluoride according to the procedure of Example 1 yielded 490 mg of the peptide after precipitation of the trifluoroacetic acid solution from ethyl ether. Then, as in Example 1, 10 mg of the peptide was treated in aqueous ethanolamine, acidified, precipitate and chromatographed to afford 3.8 mg of the title peptide.
FAB MS: 3669.1 Da (parent + H predicted: 3668.83 Da)

### EXAMPLE 5

### H₂N-R-COOH wherein R is a derivative of His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 2) where the derivative is N-terminal (N-alpha) succinoylated and the epsilon amine group of both Lys residues are succinoylated

To a solution of 800 µg (0.24 µmoles) of insulinotropin (SEQUENCE ID NO: 2) in 800 µl of dimethylformamide (DMF) was added sequentially 200 µg (2.0 µmoles) of succinic anhydride in 20 µl of DMF and 200 µg (2.0 µmoles) of triethylamine in 10 µl of DMF. The clear solution was stirred for three hours at ambient temperature, affording a single major product by reverse phase HPLC analysis, according to the method of Example 1, in approximately 90% yield (based on HPLC peak areas). The product was added to 10 ml of water, acidified with trifluoroacetic acid to pH 2 and lyophilized to dryness. The dried lyophilizate was taken up in HPLC mobile phase and purified by preparative reverse phase HPLC, according to the method of Example 1, affording a homogeneous product shown by Plasma Desorption mass spectral analysis to be tri-succinoyl substituted insulinotropin. Expected Mass (M + H): 3656.68 Da, Found: 3658.2 Da.

### EXAMPLE 6

### H₂N-R-COOH wherein R is a derivative of His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly(SEQUENCE ID NO:2) where the derivative is hexa- or hepta-succinoylated

According to the method of Example 5, 100 µg (0.03 µmoles) instead of 800 µg (0.24 µmoles) of insulinotropin (SEQUENCE ID NO: 2) was reacted according to all of the other reactive amounts of Example 5 to afforded a new product after stirring 16 hours at ambient temperature (as evidenced by HPLC analysis). The product, isolated according to the method of Example 5, was shown by ES-MS mass spectral analysis to be a mixture of hexa- and hepta-succinoylated insulinotropin. Expected Mass for hexa-succinoylated insulinotropin: 3955.68 Da, Found: 3955.9 Da. Expected Mass for hepta-succinoylated insulinotropin: 4055.68 Da, Found: 4055.9 Da.

### EXAMPLE 7

### H₂N-R-COOH wherein R is a derivative of His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 2) where the derivative is de-amidated Gln resulting in Glu

One hundred micrograms (100 µg) of insulinotropin (SEQUENCE ID NO: 2) was dissolved in a solution of 50 µl of DMF containing 20 µl of aqueous tricine buffer (pH 8.75) and stirred at 37°C overnight. HPLC according to the method of Example 1 showed the presence of a new peak which was shown to co-elute with the product produced by total synthesis according to Example 2.

## Claims

1. A derivative of a polypeptide comprising the primary structure
H₂N-W-COOH
wherein W is an amino acid sequence selected from the group consisting of
His-Asp-Glu-Phe-Glu-Arg-His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 1) and
His-Asp-Glu-Phe-Glu-Arg-His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg (SEQUENCE ID NO: 6);
and the pharmaceutically-acceptable salts thereof wherein the derivative has a pl of about 4.0 or less, or a pl of about 7.0 or greater and which derivative when processed in a mammal results in a polypeptide derivative having an insulinotropic activity.

2. A derivative of a polypeptide according to claim 1 comprising the primary structure
H₂N-W-(X)ₘ-(Y)ₙ-Z
and the pharmaceutically-acceptable salts thereof wherein m is zero or one; n is zero or one; X is a basic or neutral L-amino acid residue; Y is a basic or neutral L-amino acid residue and Z is CO₂R¹ or CONR²R³ wherein R¹ is H or (C₁-C₆) straight or branched-chain alkyl when
(a) m is one, n is zero and X is a basic L-amino acid residue, or
(b) m is zero, n is one and Y is a basic L-amino acid residue, or
(c) m and n are both one and one or both of X and Y are a basic L-amino acid residue;
R¹ is (C₁-C₆)straight or branched-chain alkyl when
(a) m and n are both zero, or
(b) m is one, n is zero and X is a neutral L-amino acid residue, or
(c) m is zero, n is one and Y is a neutral L-amino acid residue, or
(d) m and n are both one and both X and Y are a neutral L-amino acid residue;
and R² and R³ are each, independently, H or (C₁-C₆)straight or branched-chain alkyl.

3. A derivative of a polypeptide comprising the primary structure
H₂N-R-COOH
wherein R is an amino acid sequence selected from the group consisting of
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 2),
Ala-Lys-Glu-Phe-Ile-Ala-Trp-Leu-Val-Lys-Gly-Arg (SEQUENCE ID NO: 3),
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly (SEQUENCE ID NO: 4) and
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys (SEQUENCE ID NO: 5);
and the pharmaceutically-acceptable salts thereof wherein the derivative has a pl of about 4.0 or less, or a pl of about 7.0 or greater and having inculinotropic activity, provided that said derivative is not a C-terminal (C₁-C₆)straight or branched-chain alkyl ester and provided further that said derivative is not a C-terminal carboxamide of the formula CONR²R³ wherein R² and R³ are each, independently, H or (C₁-C₆)straight or branched-chain alkyl.

4. A derivative of a polypeptide according to claim 3 comprising the primary structure.
H₂N-R-X-(Y)ₙ-Z
and the pharmaceutically-acceptable salts thereof wherein n is zero or one; X is a basic or neural L-amino acid residue; Y is a basic or neutral L-amino acid residue; and Z is CO₂R¹ or CONR²R³ wherein R¹ is H or (C₁-C₆) straight or branched-chain alkyl when
(a) n is zero and X is a basic L-amino acid residue, or
(b) n is one and one or both of X and Y are basic L-amino acid residue;
R¹ is (C₁-C₆)straight or branched-chain alkyl when
(a) n is zero and X is a neutral L-amino acid residue, or
(b) n is one and both X and Y are a neutral L-amino acid residue;
and R² and R³ are each, independently, H or (C₁-C₆)straight or branched-chain alkyl.

5. A derivative of a polypeptide according to claim 4 and the pharmaceutically-acceptable salts thereof wherein R is
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 2).

6. A derivative of a polypeptide according to claim 5 and the pharmaceutically-acceptable salts thereof wherein n is zero and X is Arg.

7. A derivative of a polypeptide according to claim 6 and the pharmaceutically-acceptable salts thereof wherein Z is CO₂R¹ and R¹ is H.

8. A derivative of a polypeptide according to claim 6 and the pharmaceutically-acceptable salts thereof wherein Z is CONR²R³ and R² and R³ are each H.

9. A derivative of a polypeptide according to claim 5 and the pharmaceutically-acceptable salts thereof wherein n is one and X and Y are each Arg.

10. A derivative of a polypeptide according to claim 9 and the pharmaceutically-acceptable salts thereof wherein Z is CO₂R¹ and R¹ is H.

11. A derivative of a polypeptide according to claim 9 and the pharmaceutically-acceptable salts thereof wherein Z is CONR²R³ and R² and R³ are each H.

12. A pharmaceutical composition comprising a derivative of a polypeptide according to any preceding claim.

13. A derivative according to any of claims 1 to 11 or a pharmaceutical composition according to claim 12, for use in medicine.

14. The use of a derivative according to any of claims 1 to 11 or a pharmaceutical composition according to claim 12 in the preparation of a medicament for treating type II diabetes.

15. The use according to claim 14 wherein the medicament is for use in iontophoretic transdermal administration.

16. A process for preparing a derivative of a polypeptide comprising the primary structure
H₂N-W-COOH
wherein W is an amino acid sequence selected from the group consisting of
His-Asp-Glu-Phe-Glu-Arg-His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 1) and
His-Asp-Glu-Phe-Glu-Arg-His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg (SEQUENCE ID NO: 6);
and the pharmaceutically-acceptable salts thereof wherein the derivative has a pl of about 4.0 or less, or a pl of about 7.0 or greater and which derivative when processed in a mammal results in polypeptide derivative having an insulinotropic activity, which process comprises preparing a derivative of said primary structure by methods known per se and, optionally, converting said derivative to a pharmaceutically-acceptable salt Thereof by methods known per se.

17. A process for preparing a derivative of a polypeptide according to claim 16 comprising the primary structure
H₂N-W-(X)ₘ-(Y)ₙ-Z
and the pharmaceutically-acceptable salts thereof wherein m is zero or one; n is zero or one; X is a basic or neutral L-amino acid residue: Y is a basic or neutral L-amino acid residue; and Z is CO₂R¹ or CONR²R³ wherein R¹ is H or (C₁-C₆) straight or branched-chain alkyl when
(a) m is one, n is zero and X is a basic L-amino acid residue, or
(b) m is zero, n is one and Y is a basic L-amino acid residue, or
(c) m and n are both one and one or both of X and Y are a basic L-amino acid residue;
R¹ is (C₁-C₆)straight or branched-chain alkyl when
(a) m and n are both zero, or
(b) m is one, n is zero and X is a neutral L-amino acid residue, or
(c) m is zero, n is one and Y is a neutral L-amino acid residue, or
(d) m and n are both one and both X and Y are a neutral L-amino acid residue;
and R² and R³ are each, independently, H or (C₁-C₆)straight or branched-chain alkyl, which process comprises preparing a derivative of said primary structure by methods known per se and, optionally, converting said derivative to a pharmaceutically-acceptable salt thereof by methods known per se.

18. A process for preparing a derivative of a polypeptide comprising the primary structure
H₂N-R-COOH
wherein R is an amino acid sequence selected from the group consisting of
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 2),
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg (SEQUENCE ID NO: 3)
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly (SEQUENCE ID NO: 4) and
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys (SEQUENCE ID NO: 5);
and the pharmaceutically-acceptable salts thereof wherein the derivative has a pl of about 4.0 or less, or a pl of about 7.0 or greater and having insulinotropic activity, provided that said derivative is not a C-terminal (C₁-C₆)straight or branched-chain alkyl ester and provided further that said derivative is not a C-terminal carboxamide of the formula, CONR²R³ wherein R² and R³ are each, independently, H or (C₁-C₆ or branched-chain alkyl, which process comprises preparing a derivative of said primary structure by methods known per se and, optionally, converting said derivative to a pharmaceutically-acceptable salt thereof by methods known per se.

19. A process for preparing a derivative of a polypeptide according to claim 18 comprising the primary structure
H₂N-R-X-(Y)ₙ-Z
and the pharmaceutically-acceptable salts thereof wherein n is zero or one; X is a basic or neutral L-amino acid residue; Y is a basic or neutral L-amino acid residue; and Z is CO₂R¹ or CONR²R³ wherein R¹ is H or (C₁-C₆) straight or branched-chain alkyl when
(a) n is zero and X is a basic L-amino acid residue, or
(b) n is one and one or both of X and Y are a basic L-amino acid residue;
R¹ is (C₁-C₆)straight or branched-chain alkyl when
(a) n is zero and X is a neutral L-amino acid residue, or
(b) n is one and both X and Y are a neutral L-amino acid residue;
end R² and R³ are each, independently, H or (C₁-C₆) straight or branched-chain alkyl, which process comprises preparing a derivative of said primary structure by methods known per se and, optionally, converting said derivative to a pharmaceutically-acceptable salt thereof by methods known per se.

20. A process according to claim 19 wherein R is
His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly (SEQUENCE ID NO: 2).

21. A process according to claim 20 wherein n is zero and X is Arg.

22. A process according to claim 21 wherein Z is CO₂R¹ and R¹ is H, or Z is CONR²R³ and R² and R³ are each H.

23. A process according to claim 20 wherein n is one and X and Y are each Arg.

24. A Process according to claim 23 wherein Z is CO₂R¹ and R¹ is H, or Z is CONR²R³ and R² and R³ are each H.

25. A process according to any one of claims 16-24 wherein said derivative if formed by one or more of the following:
(a) reacting the primary structure with a (C₁-C₆)alkanol in the presence of a catalytic acid to produce a C -terminal (C₁-C₆) alkyl ester thereof;
(b) reacting the primary structure with a(C₁-C₆) alkanol in the presence of a catalytic acid to produce a (C₁-C₆) alkyl ester of the Asp and/or Glu residues thereof;
(c) synthesizing a carboxamide derivative of the primary structure by solid phase peptide synthesis;
(d) deamidating the gln residue thereof;
(e) alkylating or amidating the free amino group at the N-terminus and/or at the epsilon group of one or more Lys residues of the primary structure, or a combination thereof;
(f) reading the primary structure with a cyclic annhydride;
(g) replacing a basic amino acid residue of the primary structure with an acidic or neutral amino acid residue; and
(h) replacing a neutral amino acid residue with an acidic amino acid residue.


SEQUENCE LISTING
(1) GENERAL INFORMATION:
(i) APPLICANT: Andrews, Glenn C.
Daumy, Gaston O.
Francoeur, Michael L.
Larson, Eric R.
Pfizer Inc, (Non-US)
(ii) TITLE OF INVENTION: GLUCAGON-LIKE PEPTIDE AND INSULINOTROPIN
DERIVATIVES
(iii) NUMBER OF SEQUENCES: 6
(iv) CORRESPONDENCE ADDRESS:
(A) ADDRESSEE: Gregg C. Benson, Pfizer Inc
(B) STREET: Eastern Point Road
(C) CITY: Groton
(D) STATE: CT
(E) COUNTRY: USA
(F) ZIP: 06340
(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25
(vi) CURRENT APPLICATION DATA:
(A) APPLICATION NUMBER:
(B) FILING DATE:
(C) CLASSIFICATION:
(vii) PRIOR APPLICATION DATA:
(A) APPLICATION NUMBER: US 07/899,073
(B) FILING DATE: 15-JUN-1992
(viii) ATTORNEY/AGENT INFORMATION:
(A) NAME: Benson, Gregg C.
(B) REGISTRATION NUMBER: 30,997
(C) REFERENCE/DOCKET NUMBER: PC8156AGCB
(ix) TELECOMMUNICATION INFORMATION:
(A) TELEPHONE: (203) 441-4901
(B) TELEFAX: (203) 441-5221

(2) INFORMATION FOR SEQ ID NO:1:
(i) SEQUENCE CHARACTERISTICS:
(A) LENGTH: 37 amino acids
(B) TYPE: amino acid
(D) TOPOLOGY: linear
(ii) MOLECULE TYPE: peptide

(xi) SEQUENCE DESCRIPTION: SEQ ID NO:1:
His Asp Glu Phe Glu Arg His Ala Glu Gly Thr Phe Thr Ser Asp Val
1               5                   10                  15
Ser Ser Tyr Leu Glu Gly Gln Ala Ala Lys Glu Phe Ile Ala Trp Leu
            20                  25                  30
Val Lys Gly Arg Gly
        35

(2) INFORMATION FOR SEQ ID NO:2:
(i) SEQUENCE CHARACTERISTICS:
(A) LENGTH: 31 amino acids
(B) TYPE: amino acid
(D) TOPOLOGY: linear
(ii) MOLECULE TYPE: peptide
(xi) SEQUENCE DESCRIPTION: SEQ ID NO:2:
His Ala Glu Gly Thr Phe Thr Ser Asp Val Ser Ser Tyr Leu Glu Gly
1               5                   10                  15
Gln Ala Ala Lys Glu Phe Ile Ala Trp Leu Val Lys Gly Arg Gly
            20                  25                  30

(2) INFORMATION FOR SEQ ID NO:3:
(i) SEQUENCE CHARACTERISTICS:
(A) LENGTH: 30 amino acids
(B) TYPE: amino acid
(D) TOPOLOGY: linear
(ii) MOLECULE TYPE: peptide
(xi) SEQUENCE DESCRIPTION: SEQ ID NO:3:
His Ala Glu Gly Thr Phe Thr Ser Asp Val Ser Ser Tyr Leu Glu Gly
1               5                   10                  15
Gln Ala Ala Lys Glu Phe Ile Ala Trp Leu Val Lys Gly Arg
            20                  25                  30

(2) INFORMATION FOR SEQ ID NO:4:
(i) SEQUENCE CHARACTERISTICS:
(A) LENGTH: 29 amino acids
(B) TYPE: amino acid
(D) TOPOLOGY: linear
(ii) MOLECULE TYPE: peptide
(xi) SEQUENCE DESCRIPTION: SEQ ID NO:4:
His Ala Glu Gly Thr Phe Thr Ser Asp Val Ser Ser Tyr Leu Glu Gly
1               5                   10                  15
Gln Ala Ala Lys Glu Phe Ile Ala Trp Leu Val Lys Gly
            20                  25

(2) INFORMATION FOR SEQ ID NO:5:
(i) SEQUENCE CHARACTERISTICS:
(A) LENGTH: 28 amino acids
(B) TYPE: amino acid
(D) TOPOLOGY: linear
(ii) MOLECULE TYPE: peptide
(xi) SEQUENCE DESCRIPTION: SEQ ID NO:5:
His Ala Glu Gly Thr Phe Thr Ser Asp Val Ser Ser Tyr Leu Glu Gly
1               5                   10                  15
Gln Ala Ala Lys Glu Phe Ile Ala Trp Leu Val Lys
            20                  25

(2) INFORMATION FOR SEQ ID NO:6:
(i) SEQUENCE CHARACTERISTICS:
(A) LENGTH: 36 amino acids
(B) TYPE: amino acid
(D) TOPOLOGY: linear
(ii) MOLECULE TYPE: peptide
(xi) SEQUENCE DESCRIPTION: SEQ ID NO:6:
His Asp Glu Phe Glu Arg His Ala Glu Gly Thr Phe Thr Ser Asp Val
1               5                   10                  15
Ser Ser Tyr Leu Glu Gly Gln Ala Ala Lys Glu Phe Ile Ala Trp Leu
            20                  25                  30
Val Lys Gly Arg
        35